(19) Europäisches Patentamt
European Patent Office
Office européen des brevets

(11) Numéro de publication: **0 022 730**
**B1**

(12) # FASCICULE DE BREVET EUROPEEN

(45) Date de publication du fascicule du brevet: **06.04.83**

(21) Numéro de dépôt: **80401067.6**

(22) Date de dépôt: **16.07.80**

(51) Int. Cl.[3]: **C 07 D 471/20,** C 07 D 519/04, A 61 K 31/475 // (C07D471/20, 221/00, 209/00, 209/00)

(54) **Nouvelle classe de composés bis-indoliques utiles comme médicaments et procédé pour leur préparation.**

(30) Priorité: **17.07.79 FR 7918453**

(43) Date de publication de la demande: **21.01.81 Bulletin 81/3**

(45) Mention de la délivrance du brevet: **06.04.83 Bulletin 83/14**

(84) Etats contractants désignés: **BE CH DE GB IT LI NL**

(56) Documents cités:
**EP-A-0 010 458**
**FR-A-1 435 519**
**FR-A-2 434 172**

**«Journal of Organic Chemistry», vol. 44, Nº 22, 1979 Columbus, Ohio, USP. Mangeney *et al.*: «A new class of antitumor compounds: 5'-nor and 5',6'-seco-derivates of vinblastine-type alkaloids», pp. 3765-68.**

(73) Titulaire: **ANVAR Agence Nationale de Valorisation de la Recherche, 43, rue Caumartin, F-75436 Paris Cedex 09 (FR)**

(72) Inventeur: **Potier, Pierre, 5, rue de la Fontaine, F-78390 Bois d'Arcy (FR)**
Inventeur: **Langlois, Nicole née Petit, 32, rue de Gometz, F-91440 Bures-sur-Yvette (FR)**
Inventeur: **Langlois, Yves, 32, rue de Gometz, F-91440 Bures-sur-Yvette (FR)**
Inventeur: **Andriamialisoa, Ratremaniaina Zo, 8 Les Amonts, F-91440 Les Ulis (FR)**
Inventeur: **Mangeney, Pierre, 10, rue de l'Ingènieur Keller, F-75015 Paris (FR)**

(74) Mandataire: **Corre, Jacques Denis Paul et al, Cabinet Regimbeau 26, Avenue Kléber, F-75116 Paris (FR)**

**EP 0 022 730 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

# Nouvelle classe de composés bisindoliques utiles comme médicaments et procédé pour leur préparation

La présente invention concerne de nouveaux composés bisindoliques et un procédé de préparation de ces composés ainsi que leur application en tant que médicaments.

Il est connu depuis un certain temps que des alcaloïdes naturels, tels que la vinblastine ou la vincristine qui répondent à la formule:

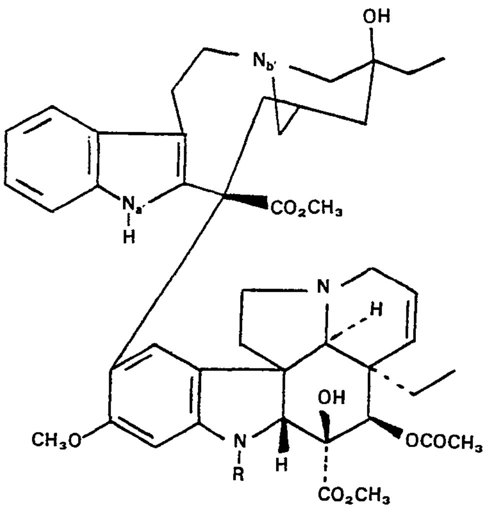

($R=CH_3$: vinblastine, $R=CHO$: vincristine)

et qui peuvent être isolés de plusieurs espèces de *Catharanthus*, en particulier de *C. roseus*, présentent des propriétés antitumorales. Compte tenu du fait que ces alcaloïdes ne sont présents qu'en très faible quantité dans la plante, on a cherché à préparer des dérivés plus actifs de ces composés (voir ainsi les BSM 5487 M, 6668 M, le brevet français N° 2218095). On a également proposé (voir les brevets français N$^{os}$ 2296418 et 2387237) des procédés de synthèse ouvrant la voie à de nouveaux composés de structure identique, mais diversement substitués.

Néanmoins, dans l'ensemble de la technique antérieure, les composés proposés conservaient toujours le squelette de base de la vinblastine.

La présente invention a pour objet des composés chimiques nouveaux possédant notamment une activité antitumorale et présentant un squelette de base différent de celui de la vinblastine.

Il s'agit de composés de formule:

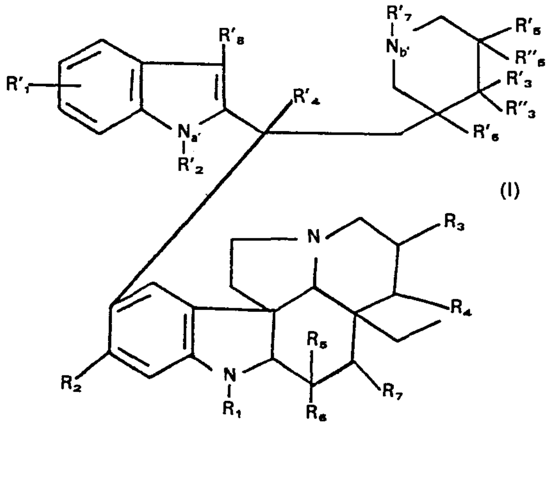

(I)

dans laquelle:

$R'_1$ représente un atome d'hydrogène ou un radical alcoxy, acyle, formyle ou halogénoacyle,

$R'_2$ représente un atome d'hydrogène ou un radical alcoyle,

$R'_3$ et $R''_3$, identiques ou différents, représentent un atome d'hydrogène ou un radical hydroxyle, alcanoyloxyle, ou bien $R'_3$ et $R''_3$ forment ensemble un groupement carbonyle, ou bien $R'_3$ et $R'_5$ forment ensemble un pont époxy ou une double liaison,

$R'_4$ représente un atome d'hydrogène ou un radical alcoyloxycarbonyle, hydroxyméthyle, alcanoyloxyméthyle ou acétamido,

$R'_5$ et $R''_5$, identiques ou différents, représentent un atome d'hydrogène ou un radical hydroxyle, alcanoyloxyle, éthyle ou hydroxy-2 éthyle,

$R'_6$ représente un atome d'hydrogène ou un radical éthyle, hydroxy-2 éthyle ou acétyle,

$R'_7$ et $R'_8$ représentent un atome d'hydrogène ou un radical méthyle, alcoxyméthylène, aryloxyméthylène, hydroxyméthylène, aminométhylène, monoalcoyl ou dialcoylaminométhylène, arylaminométhylène, hydroxyéthylaminométhylène, alcoylthiométhylène, arylthiométhylène, cyanométhylène, acylméthylène, sous réserve que $R'_7$ et $R'_8$ ne représentent pas ensemble le radical hydroxyméthylène,

$R_1$ représente un atome d'hydrogène ou un radical alcoyle, formyle ou acyle,

$R_2$ représente un atome d'hydrogène ou un radical alcoxy,

$R_3$ représente un atome d'hydrogène ou un radical hydroxyle ou alcanoyloxyle, ou bien $R_3$ et $R_4$ forment ensemble un pont époxy ou une double liaison,

$R_4$ représente un atome d'hydrogène ou un radical hydroxyle, alcanoyloxyle, ou bien $R_4$ et $R_5$ forment ensemble un pont époxy,

$R_6$ représente un radical alcoyloxycarbonyle, hydrazido, acétamido, hydroxyméthyle ou alcanoyloxyméthyle,

$R_5$ et $R_7$ représentent un atome d'hydrogène ou un radical hydroxyle ou alcanoyloxyle,

ainsi que leurs sels d'addition avec les acides et leurs sels d'ammonium quaternaires.

Les radicaux alcoyles mentionnés dans la présente description sont de préférence des radicaux alcoyles inférieurs droits ou ramifiés ayant de 1 à 5 atomes de carbone, comme les radicaux méthyl et éthyl.

Les radicaux alcoxy mentionnés dans la présente description sont de préférence des radicaux alcoxy inférieurs correspondant aux radicaux alcoyles précédents, c'est-à-dire par exemple les radicaux méthoxy et éthoxy.

Les radicaux acyles cités dans la présente description sont par exemple les radicaux acyles provenant des acides carboxyliques inférieurs, saturés ou non saturés, tels que les radicaux acétyl ou propionyl.

De même, les radicaux alcanoyloxy sont de préférence des radicaux correspondant aux radicaux acyles précédents, comme le radical acétyloxy.

Les radicaux alcoyloxycarbonyle sont de préférence des radicaux dans lesquels la partie alcoyle correspond à la définition préférée donnée précédemment, par exemple le radical méthoxycarbonyle.

Les radicaux aryles mentionnés dans la présente description sont de préférence des radicaux aryles monocycliques contenant 5 ou 6 atomes dans le cycle, et en particulier le radical phényle.

Parmi les sels d'addition et les sels d'ammonium quaternaires, on préparera de préférence les sels non toxiques pharmaceutiquement acceptables, tels que les acides inorganiques comme l'acide chlorhydrique, ou d'acides organiques comme l'acide acétique, l'acide propionique, l'acide succinique ou l'acide tartrique.

L'invention concerne plus particulièrement les composés de formule Ia:

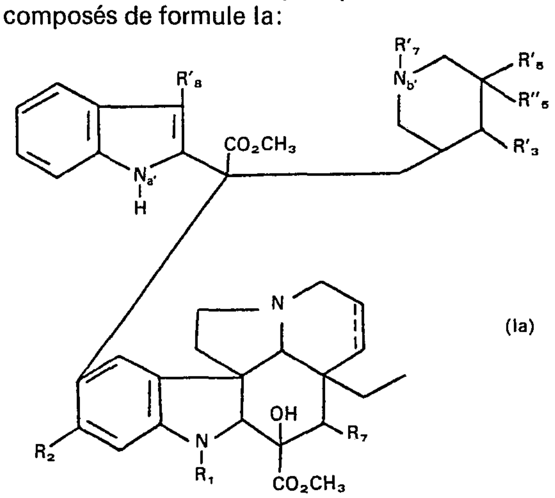

(Ia)

dans laquelle:

$R'_3$ représente un atome d'hydrogène ou un radical hydroxy,

$R'_5$ représente un atome d'hydrogène ou un radical hydroxy,

$R'_3$ et $R_5$ représentent ensemble un pont époxy ou une double liaison,

$R''_5$ représente un atome d'hydrogène ou un radical éthyle,

$R'_7$ et $R'_8$ représentent un atome d'hydrogène ou un radical méthyle, alcoxyméthylène, aryloxyméthylène, monoalcoyle ou dialcoyleaminométhylène, arylaminométhylène, hydroxyéthylaminoéthylène, alcoylthiométhylène, arylthiométhylène, cyanométhylène, acylméthylène,

$R_1$ représente un atome d'hydrogène, un radical alcoyle, formyle ou acyle,

$R_2$ représente un atome d'hydrogène ou un radical méthoxy,

$R_7$ représente un radical alcanoyloxyle,

la ligne pointillée représente une double liaison éventuelle,

ainsi que les sels correspondants.

La présente invention concerne également un procédé Ⓐ de préparation de composés de formule I, caractérisé en ce que:

a) l'on fait réagir un composé de formule II:

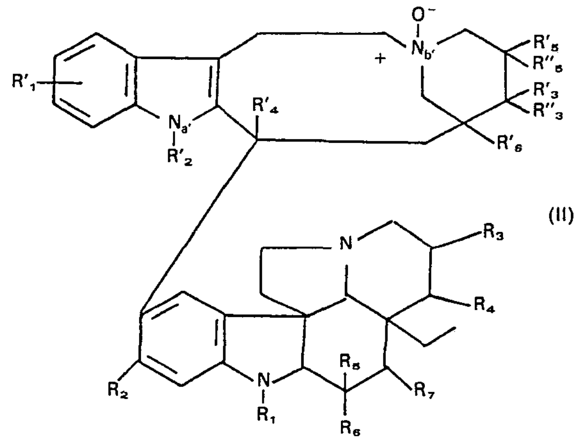

(II)

dans laquelle les substituants ont la signification donnée précédemment, en présence d'un réactif formateur d'ion immonium,

b) l'on traite l'ion immonium obtenu par un ou plusieurs réactifs nucléophiles et sépare le composé de formule I.

Parmi les agents formateurs d'ion immonium, on utilise de préférence les halogénures, anhydrides ou sels d'acides organiques ou inorganiques, en particulier d'acides carboxyliques halogénés (en particulier fluorés) ou non.

Les réactifs formateurs d'ion immonium sont par exemple l'anhydride d'acide acétique ou trifluoroacétique.

L'étape a est conduite de préférence dans un solvant organique anhydre tel qu'un solvant chloré, chlorure de méthylène, dichloroéthane ou chloroforme.

La réaction a est conduite de préférence à une température comprise entre −5 et +5°C, par exemple 0°C.

L'étape b est mise en œuvre de préférence dans un solvant, en particulier un solvant organique comme le méthanol, l'éthanol, le tétrahydrofuranne, le dioxanne, le diméthylformamide, le diméthylsulfoxyde, l'hexaméthylphosphorotriamide ou l'acétonitrile, le chloroforme ou le dichlorométhane.

L'étape b peut être conduite à température ambiante.

Pour la préparation des composés de formule I dans lesquels $R'_7$ et $R'_8$ sont de la forme $R'_7 = -CH_2-R''_7$ et $R'_8 = -CH_2-R''_8$, on utilisera un ou plusieurs réactifs nucléophiles fixant les radicaux $R''_7$ et $R''_8$ en position 5' et 6' de l'ion immonium. (La préparation de ces ions immonium est également décrite dans la demande de brevet français N° 2434172 au nom de la titulaire.)

Le solvant de l'étape b peut être lui-même un réactif nucléophile tel que le méthanol ou l'éthanol pour fixer des radicaux $R''_7$ et/ou $R''_8$ alcoxy correspondants.

Comme autres nucléophiles, on peut utiliser les ions hydrures obtenus par action d'un borohydrure alcalin, les ions cyanures, les ions thiolates, thiophénates ou phénates, une amine ou l'ammoniaque ou un acide carboxylique suivant le type de substituant que l'on désire fixer.

Dans le cas où l'un des substituants $R'_7$ et/ou $R'_8$ est l'atome d'hydrogène, on traite l'ion immonium en solution par un agent alcalin tel que le carbonate de sodium dans l'eau.

Lorsque l'on utilise un solvant différent dans l'étape a et dans l'étape b, il est nécessaire de chasser le solvant du mélange réactionnel de l'étape a avant la mise en œuvre de l'étape b; on peut par exemple le distiller.

Il est possible également de conduire l'étape a et l'étape b dans un même solvant; dans ce cas, l'étape a est conduite dans le solvant anhydre et on rajoute le réactif nucléophile pour la mise en œuvre de l'étape b.

La séparation du composé I peut être conduite par tout moyen connu de séparation; par exemple, on peut extraire le produit de formule I par un solvant chloré tel que le chloroforme, puis séparer le composé de formule I de la phase chloroformique par chromatographie préparative.

La séparation conduit à l'obtention d'un sous-produit qui, par réduction, donne un composé de formule III mentionné ci-dessous qui peut être recyclé.

Les composés de formule II sont connus (voir les brevets cités précédemment) ou peuvent être obtenus à partir de composés de formule III:

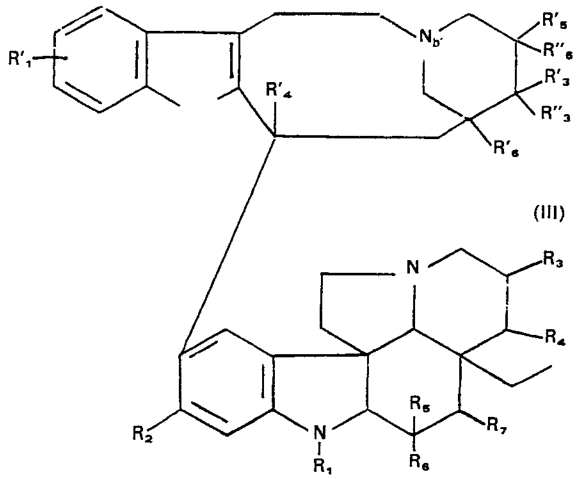

(III)

par des procédés connus dans ce domaine, en particulier par oxydation, par exemple à l'aide d'acides perbenzoïques dans un solvant, notamment l'acide m-chloroperbenzoïque, dans le chloroforme ou dans le chlorure de méthylène.

Les composés de formule III sont connus ou peuvent être préparés par des procédés décrits dans les brevets cités précédemment.

Procédé (A)

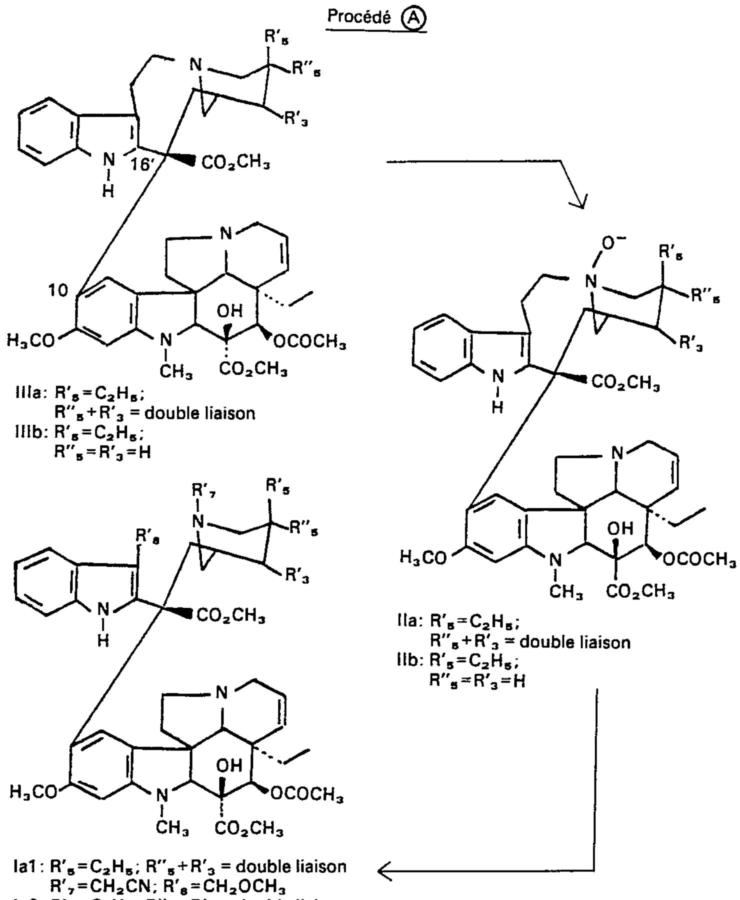

IIIa: $R'_5=C_2H_5$; $R''_5+R'_3$ = double liaison
IIIb: $R'_5=C_2H_5$; $R''_5=R'_3=H$

IIa: $R'_5=C_2H_5$; $R''_5+R'_3$ = double liaison
IIb: $R'_5=C_2H_5$; $R''_5=R'_3=H$

Ia1: $R'_5=C_2H_5$; $R''_5+R'_3$ = double liaison $R'_7=CH_2CN$; $R'_8=CH_2OCH_3$
Ia2: $R'_5=C_2H_5$; $R''_5=R'_3$ = double liaison $R'_7=CH_3$; $R'_8=CH_3$
Ia3: $R'_5=C_2H_5$; $R''_5+R'_3$ = double liaison $R'_7=CH_3$; $R'_8=CH_2OCH_3$
Ia4: $R'_5=C_2H_5$; $R''_5=R'_3=H$; $R'_7=CH_2CH$ $R'_8=CH_2OCH_3$
Ia5: $R'_5=C_2H_5$; $R''_5=R'_3=H$; $R'_7=H$ $R'_8=CH_2OH$

La présente invention concerne également un procédé Ⓑ de préparation de composés de formule I, caractérisé en ce que l'on fait réagir un composé de formule générale IV:

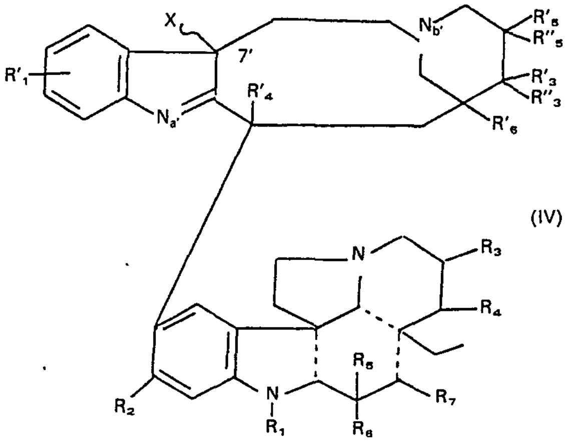

(IV)

dans laquelle les substituants ont la signification donnée précédemment et X représente un halogène, un radical hydroxy, hydroperoxy ou alcanoyloxy, avec un ou plusieurs réactifs nucléophiles, la réaction pouvant être conduite en solution dans l'eau ou dans un solvant organique.

Le procédé selon la présente invention peut être mis en œuvre sans catalyseur, mais la réaction peut être catalysée par des ions $Ag^+$ provenant de sels d'argent tels que le perchlorate, le tétrafluoroborate, le trifluoroacétate et l'acétate d'argent ou bien un réactif acide, par exemple un acide inorganique tel que l'acide chlorhydrique.

Le solvant utilisé peut être un solvant organique en présence ou non d'eau. Parmi les solvants organiques qui peuvent être utilisés, on peut citer le méthanol, l'éthanol, le tétrahydrofuranne, le chloroforme, le dichlorométhane, le dioxanne, le diméthylformamide, le diméthylsulfoxyde, l'hexaméthylphosphotriamide ou l'acétonitrile, le chloroforme ou le dichlorométhane.

La température de la réaction est comprise de préférence entre 0 et 100°C.

Le nucléophile utilisé peut être choisi parmi les ions hydrures obtenus par action d'un borohydrure alcalin, les ions cyanures, les ions thiolates, thiophénates ou phénates, une amine, un alcool ou l'ammoniaque. Le solvant peut être lui-même le nucléophile, tel le méthanol ou l'éthanol.

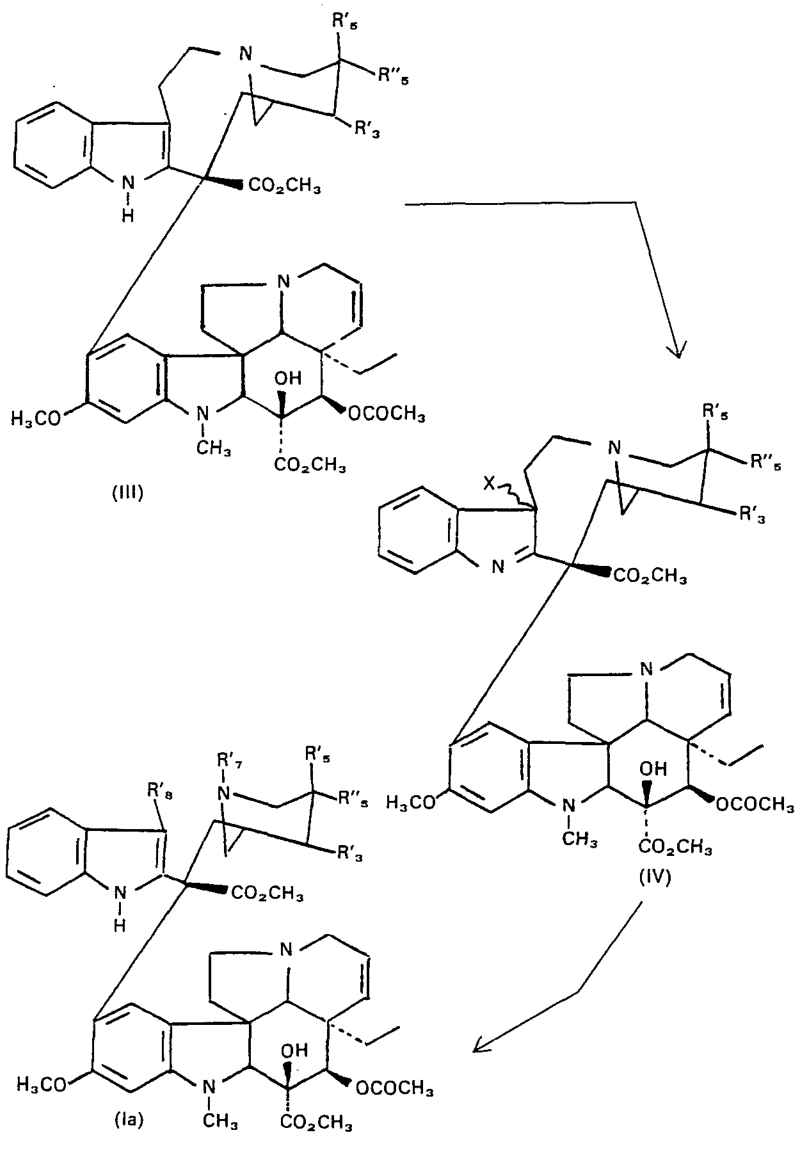

(III)

(IV)

(Ia)

La séparation de I peut être conduite comme dans le procédé Ⓐ.

Les composés de formule IV sont connus (brevet français N° 79.02981) et peuvent être obtenus à partir de composés de formule III, eux-mêmes décrits dans les brevets précédemment cités.

D'autres caractéristiques de mise en œuvre du procédé Ⓐ et du procédé Ⓑ selon la présente invention pourront être déduites des exemples suivants:

*Exemple 1*

*Préparation du $N_{b'}$-oxyde de $\Delta^{15'}$ déshydroxy-20' vincaleucoblastine (IIa) (anhydrovinblastine)*

A une solution de $\Delta^{15'}$ déshydroxy-20' vincaleucoblastine (IIIa) (748 mg; 0,94 mmol) dans 10 ml de chlorure de méthylène à 0°C sous agitation et sous argon, on ajoute en une seule fois 180 mg (1,04 mmol) d'acide métachloroperbenzoïque. On laisse en contact 30 min à 0°C. Puis on reprend le milieu réactionnel par 100 ml de chloroforme et on lave la phase organique 4 fois avec 3 ml d'une solution aqueuse de bicarbonate de sodium à 40 g/l. La phase organique est ensuite lavée à neutralité par de l'eau saturée de chlorure de sodium (3×10 ml), séchée par du sulfate de sodium, filtrée et évaporée sous vide pour fournir 712 mg de $N_{b'}$-oxyde de $\Delta^{15'}$ déshydroxy-20' vincaleucoblastine (Rdt: 93%).

U.V. (λ nm, ε): 213, 268, 289, 293, 310. RMN$^1$H (400 MHz; δ = 0 ppm; TMS; J: Hz) 9,50 (s, 1H, $C_{16}$-OH); 8,19 (s, 1H, Na'-H); 7,70 (d, 1H, J≃7,5; $C_9$-H ou $C_{12'}$-H); 7,19 et 7,14 ($C_{10'}$-H); 7,19 et 7,14 ($C_{10'}$-H et $C_{11'}$-H); 7,07 (d, J≃7,5, $C_{12'}$-H ou $C_{9'}$-H); 6,43 (s, 1H, $C_9$-H); 6,11 (s, 1H, $C_{12}$-H); 5,84 (dd, 1H, $J_{14,15}$≃10 Hz et $J_{3,14}$ ≃4,5, $C_{14}$-H); 5,44 (1H, $C_{15'}$-H); 5,41 (s, 1H, $C_{17}$-H); 5,27 (d, 1H, $J_{14,15}$≃10 Hz, $C_{15}$-H); 4,51 (m, 2H); 4,32 (d, 1H) et 3,98 (d, 1H, $N_{b'}$−CH); 3,84 (s, 3H); 3,78 (s, 3H) et 3,69 (s, 3H, $C_{11}$-$OCH_3$, $C_{16}CO_2CH_3$ et $C_{16'}CO_2CH_3$); 2,62 (s, 3H, Na−$CH_3$); 2,09 (s, 3H, $OCOCH_3$); 1,06 (t, 3H, $J_{18',19'}$ = 7 Hz, $C_{18'}$-H et 0,64 (t, 3H, $J_{18,19}$≃7 Hz, $C_{18}$-H). S.M.: 808, 806, 792 (M-16), 777, 761, 733, 669, 633, 631, 612, 611, 610, 510, 469, 282, 222, 200, 193, 144, 136, 135, 122, 121 (pic de base), 108, 107.

*Préparation de la séco-5',6' cyano-5' méthoxy-6' anhydrovinblastine Ia1*

Le $N_{b'}$-oxyde d'anhydrovinblastine IIa (145 mg; 0,18 mmol) en solution dans le dichlorométhane sec est traité durant 1 h sous argon par de l'anhydride trifluoroacétique (0,12 ml; 0,83 mmol). Après évaporation du solvant sans chauffer, le résidu est dissous dans une solution saturée de cyanure de potassium dans le méthanol. Cette solution est agitée pendant 2 h à 20°C, jetée dans une solution aqueuse saturée de chlorure de sodium et extraite par du chloroforme (3×50 ml). Après séchage sur sulfate de sodium, filtration et évaporation sous vide de la solution chloroformique, le résidu sec est purifié par chromatographie préparative sur plaque (éluant: $CHCl_3$/MeOH: 93/7) et le composé Ia1 est obtenu (82 mg, 54%).

*Composé Ia1:* $[\alpha]_D^{20}$ = +7° (EtOH, C=0,6). I.R.: 3410, 2960, 1740, 1620. U.V.: 222 (37 000), 264 (11 200), 296 (8000), 318 sh (5600). S.M. m/e: 863 ($M^{+\cdot}$+14), 849 ($M^{+\cdot}$), 819, 817, 790, 658, 656, 598, 379, 282, 258, 244, 222, 161, 135, 122, 121, 10). R.M.N. (240 MHz): 7,61 et 7,37 (d, J=7 et d, J=7,5, 2H, H aromatique indolique), 7,14 (m, 2H, H aromatique indolique), 7,12 et 5,97 (2s, 2H, $C_9$-H et $C_{12}$-H), 5,91 (dd, $J_{14,15}$ = 9,5, $J_{3,14}$ = 3,5, 1H, $C_{14}$-H), 5,58 (s, 1H, $C_{17}$-H), 5,40 (d, $J_{14,15}$ = 9,5, 1H, $C_{15}$-H), 4,87 (m, 1H, $C_{15'}$-H), 4,29 et 4,19 (2d, $J_{6'a,6'b}$ = 12, 2H, $C_{6'}$-$H_2$), 3,75, 3,61 et 3,36 (3s, 9H, $C_{11}$-$OCH_3$, $C_{16}CO_2CH_3$ et $C_{16'}CO_2CH_3$), 3,07 (s, 3H, $CH_2$-$OCH_3$), 0,84 et 0,76 (2t, 6H, $C_{18}$-$H_3$ et $C_{18'}$-$H_3$).

*Exemple 2*

*Préparation de la séco-5',6' anhydrovinblastine Ia2*

Le $N_{b'}$-oxyde d'anhydrovinblastine IIa (98 mg; 0,12 mmol) en solution dans le dichlorométhane sec est traité durant 1 h à 0°C et sous argon par l'anhydride trifluoroacétique (0,08 ml; 0,5 mmol). Après évaporation du solvant sans chauffer et sous vide, le résidu est dissous dans du méthanol (2 ml) et réduit par du cyanoborohydrure de sodium (7 mg) pendant 30 min. Après traitement comme ci-dessus, le résidu est purifié par chromatographie sur plaque préparative (éluant: $CHCl_3$/MeOH: 90/10). L'anhydrovinblastine IIIa (24 mg; 24%) et le composé Ia2 (33 mg; 35%) sont isolés.

*Composé Ia2:* $[\alpha]_D^{20}$ = +19° (EtOH, C=0,5). I.R. = 3460, 2960, 1740, 1610. U.V.: 218 (13000), 263 (5980), 288 (4700), 296 (4700). D.C.: 248 (+), 215 (+), 200 (−). S.M. m/e: 808, 794 ($M^{+\cdot}$), 735, 657, 635, 598, 527, 282, 188, 152, 135, 122. R.M.N. (240 MHz): 7,41 et 7,29 (2d, J=7,5, 2H, H aromatique indolique), 7,06 (m, 2H, H aromatique indolique), 6,90 et 6,00 (2s, 2H, $C_9$-H et $C_{12}$-H), 5,85 (dd, $J_{14,15}$ = 9,5 et $J_{3,14}$ = 3,5, 1H, $C_{14}$-H), 5,46 (s, 1H, $C_{17}$-H), 5,32 (d, $J_{14,15}$ = 9,5, 1H, $C_{15}$-H), 4,90 (s, large, 1H, $C_{15'}$-H), 3,76, 3,61 et 3,53 (s, 9H, $C_{11}$-$OCH_3$, $C_{16}CO_2CH_3$ et $C_{16'}CO_2CH_3$), 2,66 (s, 3H, $N_a$-$CH_3$), 2,24 (s, 3H, $N_{b'}$-$CH_3$), 2,08 (s, 3H, $OCOCH_3$), 1,95 (s, 3H, $C_{7'}$-$CH_3$), 0,87 et 0,63 (2t, $J_{18,19}$ = 7,5, 6H, $C_{18'}$-$H_3$ et $C_{18}$-$H_3$).

*Exemple 3*

*Préparation de la séco-5',6' méthoxy-6' anhydrovinblastine Ia3*

Le $N_{b'}$-oxyde d'anhydrovinblastine IIa est traité comme pour la préparation du composé Ia2 et réduit par un excès de borohydrure de sodium dans le méthanol. Après traitement comme ci-dessus et purification par chromatographie sur plaque préparative (éluant: $CHCl_3$/MeOH: 90/10), le composé Ia3 (30%) et l'anhydrovinblastine IIIa (25%) sont obtenus.

*Composé Ia3:* $[\alpha]_D^{20}$ = −7,3° (EtOH, C=0,6). I.R.: 3405, 2950, 1745, 1620. U.V.: 220 (37 100), 263 (11 400), 284 (9100), 292 (8500), 314

(5700). D.C.: 257 (+), 215 (+), 200 (−). S.M. m/e: 838, 824 ($M^{+\cdot}$), 808, 794, 793, 792, 748, 734, 656, 598 (100%), 336, 282, 152, 135, 122. R.M.N. (240 MHz): 7,41 et 7,29 (2d, J=7,5, 2H, H aromatique indolique), 7,03 (m, 2H, H aromatique indolique), 6,98 et 6,00 (2s, 2H, $C_9$-H et $C_{12}$-H), 5,90 (dd, $J_{14,15}$ = 9,5 et $J_{3,14}$ = 3,5, 1H, $C_{14}$-H), 5,54 (s, 1H, $C_{17}$-H), 5,40 (d, $J_{14,15}$ = 9,5, 1H, $C_{15}$-H), 5,02 (s, large, 1H, $C_{15'}$-H), 4,39 (dd, $J_{6'a,6'b}$ = 11, 2H, $C_{6'}$-$H_2$), 3,75, 3,62 et 3,48 (3s, 9H, $C_{11}$-$OCH_3$, $C_{16}CO_2CH_3$ et $C_{16'}CO_2CH_3$), 3,10 (s, 3H, $C_{6'}$-$OCH_3$), 2,71 (s, 3H, $N_a$−$CH_3$), 2,38 (s, 3H, $N_{b'}$−$CH_3$), 2,16 (s, 3H, $OCOCH_3$), 0,98 et 0,85 (2t, $J_{18,19}$ = 7,5, 6H, $C_{18'}$-$H_3$ et $C_{18}$-$H_3$).

*Exemple 4*

*Préparation du $N_{b'}$-oxyde de déoxy-20′ leurosidine IIb*

A une solution de déoxy-20′ leurosidine IIIb (45 mg; 0,056 mmol) dans le dichlorométhane sec (1 ml) on ajoute à 0° C l'acide p-nitroperbenzoïque (13 mg; 0,07 mmol). Après 10 min, le milieu réactionnel est repris par une solution aqueuse de $Na_2CO_3$ (1 ml; C=40 g/l) et extrait 3 fois avec du chloroforme. Après traitement comme ci-dessus et purification par chromatographie sur plaque préparative (éluant: $CHCl_3$/MeOH: 90/10), le $N_{b'}$-oxyde de déoxy-20′ leurosidine est isolé (38 mg; 83%).

U.V.: 228, 268, 286, 296, 312. S.M. m/e: 822, 808, 794, 792, 612, 610, 468, 282, 138, 135 (100%), 124, 122, 121. R.M.N. (60 MHz): 9,5 (s, 1H, $C_{16}$-OH), 7,9 (s large, 1H, $N_{a'}$-H), 7,3-6,8 (m, 4H aromatiques), 6,3 et 6,0 (2s, 2H, $C_9$-H et $C_{12}$-H), 5,6 (m, 1H, $C_{14}$-H), 5,3 (s+m, 2H, $C_{17}$-H et $C_{15}$-H), 3,7 et 3,5 (2s, 9H, $OCH_3$, $C_{16}$-$CO_2CH_3$ et $C_{16'}$-$CO_2CH_3$), 2,7 (s, 3H, $N_a$-$CH_3$), 2,0 (s, 3H, $OCOCH_3$), 0,8 (m, 6H, $C_{18}$-$H_3$ et $C_{18'}$-$H_3$).

*Préparation de la séco-5′,6′ cyano-5′ méthoxy-6′ déoxy-20′ leurosidine Ia4*

Le $N_{b'}$-oxyde de déoxy-20′ leurosidine (37 mg; 0,045 mmol) en solution dans le dichlorométhane sec (0,6 ml) est traité, à 0° C sous argon, par l'anhydride trifluoroacétique (0,03 ml; 0,21 mmol). Après 1 h, le milieu réactionnel est évaporé sous vide et dissous dans le méthanol anhydre (2,5 ml). Le cyanure de potassium (25 mg) est ajouté à cette solution à 0° C. Après 30 min, le milieu réactionnel est repris par de l'eau et extrait par du chloroforme. Après traitement habituel, le résidu (38 mg) est purifié par chromatographie préparative sur plaque (éluant: $CHCl_3$/MeOH: 93/7) et le composé Ia4 est isolé (19 mg; 51%).

I.R.: 3300, 2950, 2300, 1740. U.V.: 220 (58000), 264 (11 800), 284 (9900), 295 (9100), 315 (sh 6000). D.C.: 295 (−3,8), 285 (−3,8), 255 (+15,2), 228 (+9,5), 205 (−22,8). R.M.N. (60 MHz): 9,8 (s, large, $N_{a'}$−H ou OH), 7,2-7,0 (m, 5H aromatiques), 6,0 (s, 1H, $C_9$-H ou $C_{12}$-H), 5,5 (s, 1H, $C_{17}$-H), 5,4 (s, 1H, $C_{15}$-H), 4,2 (s large, 2H, $C_{5'}$-H ou $C_{6'}$-H), 3,8, 3,65, 3,45, 3,05 (4s, 12H, $OCH_3$, $C_{16}$-$CO_2CH_3$, $C_{16'}$-$CO_2CH_3$, $C_{6'}$-$OCH_3$), 2,65 (s, 3H, $N_a$-$CH_3$), 2,05 (s, 3H, $OCOCH_3$), 0,65 (m, 6H, $C_{18}$-$H_3$ et $C_{18'}$-$H_3$). S.M. m/e: 851 ($M^{+\cdot}$), 821, 819, 690, 660, 282 (100%), 222, 163 (100%), 138 (100%), 136, 135 (100%), 124, 122, 121.

*Exemple 5*

*Préparation de la séco-5′,6′ déméthyl-5′ hydroxy-6′ déoxy-20′ leurosidine Ia5:*

A une solution de $N_{b'}$-oxyde de déoxy-20′ leurosidine (70 mg; 0,09 mmol) dans le dichlorométhane sec (0,67 ml) sous agitation et sous argon, on ajoute l'anhydride trifluoroacétique (0,065 ml; 0,45 mmol). Après 1 h, le milieu réactionnel est évaporé sous vide et le résidu est dissous dans le tétrahydrofuranne (5 ml). Une solution aqueuse de $CO_3Na_2$ (40 g/l; 0,012 ml) est ajoutée et le milieu réactionnel est agité une nuit à température ordinaire. Après extraction par du $CHCl_3$ et traitement habituel, le résidu est purifié par chromatographie préparative sur plaque (éluant: $CHCl_3$/MeOH: 90/10), le composé Ia5 est isolé (16 mg; 23%).

I.R.: 3400, 2950, 1740. U.V.: 222 (50 000), 265 (14 000), 285 (12 000), 292 (11 200), 310 (sh 7000). D.C.: 295 (−4,0), 287 (−4,0), 260 (+18,3), 225 (+16,6), 215 (−18,0). S.M. m/e: 810, 796, 794, 764, 750, 738, 736, 598, 577, 522, 480, 282, 144, 138 (100%), 135 (100%), 125, 124 (100%), 122, 121. R.M.N. (250 MHz): 8,70 (s, 1H, $N_{a'}$−H ou OH), 7,55-7,0 (m, 4H aromatiques), 7,02 et 5,97 (2s, 2H, $C_9$-H et $C_{12}$-H), 5,91 (m, 1H, $C_{14}$-H), 5,49 (s, 1H, $C_{17}$-H), 5,34 (d, $J_{14,15}$ = 7,5, 1H, $C_{15}$-H), 4,28 (s large, 2H, $C_{6'}$-H), 3,79, 3,66, 3,42 (3s, 9H, $OCH_3$, $C_{16}CO_2CH_3$, $C_{16'}CO_2CH_3$), 2,70 (s, 3H, $N_a$−$CH_3$), 2,12 (s, 3H, $OCOCH_3$), 0,70 et 0,52 (2t, 6H, J=7, $C_{18}$-$H_3$ et $C_{18'}$-$H_3$).

*Exemple 6*

*Préparation de la séco-5′,6′ méthoxy-6′ anhydrovinblastine Ia3 (Procédé Ⓑ)*

A une solution de 66 mg (0,08 mmol) de chloro-7′ indoléine d'anhydrovinblastine préparée comme décrit dans le brevet N° 79.02981 (6.02.79) (1er certificat additif à la demande de brevet N° 78.24568 du 24.08.78) dans 7 $cm^3$ de THF anhydre, on ajoute sous argon 18 mg d'$AgBF_4$ et le mélange est agité à 40° C pendant 5 h. Le solvant est éliminé par évaporation sous pression réduite. Après addition de 2 $cm^3$ de méthanol et réduction par un excès de borohydrure de sodium et traitements habituels, on isole 34 mg (50%) du dimère Ia3.

Les composés de formule I selon la présente invention présentent des propriétés antitumorales et peuvent donc être utilisés dans le traitement de diverses affections tumorales, telles les leucémies.

La présente invention concerne donc également, à titre de médicaments nouveaux, les composés de formule I ainsi que les compositions pharmaceutiques les contenant.

On donne ci-après les résultats des études pharmacologiques *in vitro* et *in vivo*.

*Inhibition de la polymérisation de la tubuline*

Le récepteur des alcaloïdes indoliques antitumoraux de type vinblastine est la tubuline.

Elle est facilement extraite du cerveau de porc où elle représente 10% des protéines solubles.

La polymérisation de la tubuline en microtubules peut être facilement suivie au moyen d'un spectrophotomètre U.V. en observant à 350 nm. La vitesse maximale de polymérisation est ainsi déterminée; celle-ci est diminuée par l'addition d'inhibiteurs du type de la vinblastine, et une concentration, $I_{50}$, diminuant de moitié la vitesse est obtenue pour chaque substance essayée. Un deuxième effet de ces produits est pris en considération: à des doses plus élevées, après inhibition totale de la polymérisation, une spiralisation de la tubuline est observée (vérifiée par microscopie électronique) et une nouvelle concentration, $S_{50}$, correspondant à l'apparition de 50% du phénomène, est déterminée.

Les résultats de chaque produit sont toujours comparés à ceux de la vinblastine utilisée comme référence.

Il a été établi que les résultats obtenus lors de l'utilisation des expériences précitées présentaient une excellente corrélation avec les résultats obtenus dans les cas de tumeurs animales. Ainsi, dans le tableau suivant, les composés présentant des valeurs de $I_{50}$ ou $S_{50}$ de l'ordre de $10^{-5}$ au moins sont thérapeutiquement intéressants.

*Résultats:*

| Composés | $I_{50}$ mol/l | $S_{50}$ mol/l |
|---|---|---|
| Vinblastine | $1{,}7\times10^{-6}$ | $9\times10^{-6}$ |
| Ia1 | $3{,}4\times10^{-6}$ | $4{,}5\times10^{-6}$ |
| Ia2 | $2{,}38\times10^{-4}$ | — |
| Ia3 | $5{,}1\times10^{-6}$ | $6{,}7\times10^{-6}$ |
| Ia4 | $6{,}4\times10^{-5}$ | — |
| Ia5 | $1{,}7\times10^{-5}$ | — |

La présente invention concerne également les compositions pharmaceutiques contenant un nouveau composé de formule I ou un de ses sels, éventuellement en association avec tout autre produit pharmaceutique compatible, pouvant être inerte ou physiologiquement actif.

Ces compositions peuvent être présentées sous toute forme appropriée à la voie d'administration prévue. La voie parentale est la voie d'administration préférentielle, notamment la voie intraveineuse.

Les compositions selon l'invention pour administration parentérale peuvent être des solutions stériles aqueuses ou non aqueuses, des suspensions ou des émulsions. Comme solvant ou véhicule, on peut employer le propylèneglycol, le polyéthylèneglycol, les huiles végétales, en particulier l'huile d'olive, les esters organiques injectables, en particulier l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent être également préparées sous forme de compositions solides stériles qui peuvent être dissoutes ou dispersées au moment de l'emploi dans de l'eau stérile ou tout autre milieu stérile injectable.

Les composés nouveaux ou leurs sels sont actifs dans le traitement des tumeurs solides ou liquides et plus particulièrement des cancers humains à des doses journalières comprises entre 10 et 20 mg/d pour un adulte.

L'exemple suivant illustre une composition selon l'invention.

*Exemple:*

On prépare une solution contenant 10 mg/cm³ de matière active en dissolvant 1 g du produit Ia dans 100 cm³ de soluté physiologique apyrogène. La solution obtenue est répartie aseptiquement en ampoules de 2 cm³ à raison de 1 cm³ par ampoule. Les ampoules sont scellées et contiennent chacune 10 mg de principe actif.

## Revendications

1. Composés de formule générale:

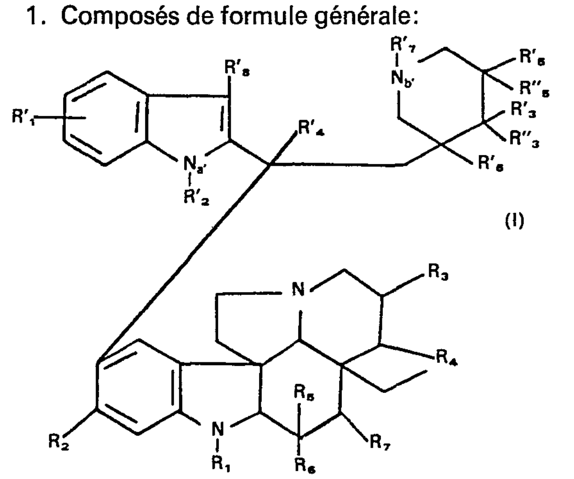

(I)

dans laquelle:

$R'_1$ représente un atome d'hydrogène ou un radical $C_1$-$C_5$ alcoxy, acétyle, propionyle ou formyle, halogénoacétyle ou halogénopropionyle,

$R'_2$ représente un atome d'hydrogène ou un radical $C_1$-$C_5$ alcoyle,

$R'_3$ et $R''_3$, identiques ou différents, représentent un atome d'hydrogène ou un radical hydroxyle, acétyloxy ou propionyloxy, ou bien $R'_3$ et $R''_3$ forment ensemble un groupement carbonyle, ou bien $R'_3$ et $R'_5$ forment ensemble un pont époxy ou une double liaison,

$R'_4$ représente un atome d'hydrogène ou un radical ($C_1$-$C_5$ alcoyl)oxycarbonyle, hydroxyméthyle, acétyloxyméthyle, propionyloxyméthyle ou acétamido,

$R'_5$ et $R''_5$, identiques ou différents, représentent un atome d'hydrogène ou un radical hydroxyle, acétyloxy, propionyloxy, éthyle ou hydroxy-2-éthyle,

$R'_6$ représente un atome d'hydrogène ou un radical éthyle, hydroxy-2-éthyle ou acétyle,

$R'_7$ et $R'_8$, identiques ou différents, représentent un atome d'hydrogène ou un radical méthyle, hydroxyméthylène, $C_2$-$C_5$ alcoxyméthylène, aryloxyméthylène, aminométhylène, monoalcoyle en $C_1$-$C_5$ et di-($C_1$-$C_5$ alcoyle)aminoéthylène, arylaminométhylène, hydroxyéthylène aminométhylène, ($C_1$-$C_5$ alcoyle)thiométhylène, arylthiométhylène, cyanométhylène, acétyl- ou propionylméthylène, sous réserve que $R'_7$ et $R'_8$ ne représentent pas ensemble le radical hydroxyméthylène, les radicaux aryles étant monocycliques et contenant 5 ou 6 atomes,

$R_1$ représente un atome d'hydrogène ou un radical $C_1$-$C_5$ alcoyle, formyle, acétyle ou propionyle,

$R_2$ représente un atome d'hydrogène ou un radical $C_1$-$C_5$ alcoxy,

$R_3$ représente un atome d'hydrogène ou un radical hydroxyle, acétyloxy ou propionyloxy, ou bien $R_3$ et $R_4$ forment ensemble un pont époxy ou une double liaison,

$R_4$ représente un atome d'hydrogène ou un radical hydroxyle, acétyloxy ou propionyloxy, ou bien $R_4$ et $R_5$ forment ensemble un pont époxy,

$R_6$ représente un radical ($C_1$-$C_5$ alcoyle)oxycarbonyle, hydrazido, acétamido, hydroxyméthyle, acétyloxyméthylène ou propionyloxyméthylène,

$R_5$ et $R_7$ représentent un atome d'hydrogène ou un radical hydroxyle, acétyloxy ou propionyloxy, ainsi que leurs sels d'addition avec les acides et leurs sels d'ammonium quaternaire.

2. Composés selon la revendication 1 de formule Ia:

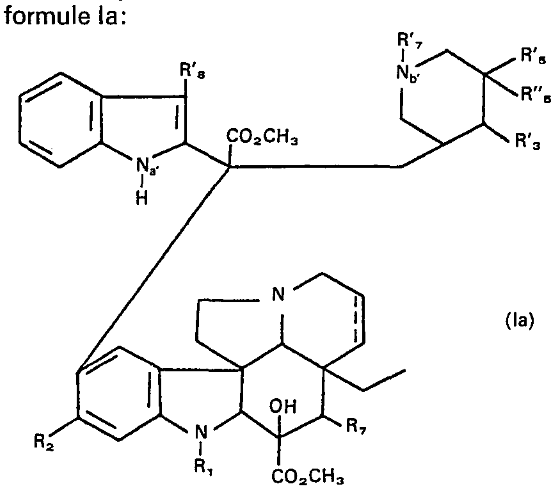

(Ia)

dans laquelle:

$R'_3$ représente un atome d'hydrogène ou un radical hydroxy,

$R'_5$ représente un atome d'hydrogène ou un radical hydroxy,

$R'_3$ et $R'_5$ représentent ensemble un pont époxy ou une double liaison,

$R''_5$ représente un atome d'hydrogène ou un radical éthyle,

$R'_7$ et $R'_8$, identiques ou différents, représentent un atome d'hydrogène ou un radical méthyle, hydroxyméthylène, ($C_1$-$C_5$ alcoxy)méthylène, aryloxyméthylène, aminométhylène, ($C_1$-$C_5$ monoalcoyl)- ou ($C_1$-$C_5$ dialcoyl)aminométhylène, ($C_1$-$C_5$ acyl)méthylène, arylaminométhylène, hydroxyéthylaminométhylène, ($C_1$-$C_5$ alcoyle)thiométhylène, arylthiométhylène, cyanométhylène, sous réserve que $R'_7$ et $R'_8$ ne représentent pas ensemble le radical hydroxyméthylène, les radicaux aryles étant monocycliques et contenant 5 ou 6 atomes,

$R_1$ représente un atome d'hydrogène ou un radical $C_1$-$C_5$ alcoyle, formyle ou acyle,

$R_2$ représente un atome d'hydrogène ou un radical $C_1$-$C_5$ alcoxy,

$R_7$ représente un radical alcanoyloxyle,

la ligne pointillée représente une double liaison éventuelle,

ainsi que les sels correspondants.

3. Les composés de formule Ia1 à Ia5:

séco-5',6' cyano-5' méthoxy-6' anhydrovinblastine,

séco-5',6' anhydrovinblastine,

séco-5',6' méthoxy-6' anhydrovinblastine,

séco-5',6' cyano-5' méthoxy-6' déoxy-20' leurosidine,

séco-5',6' diméthyl-5' hydroxy-6' déoxy-20' leurosidine.

4. Un procédé de préparation des composés selon la revendication 1, caractérisé en ce que:

a) l'on fait réagr un composé de formule II:

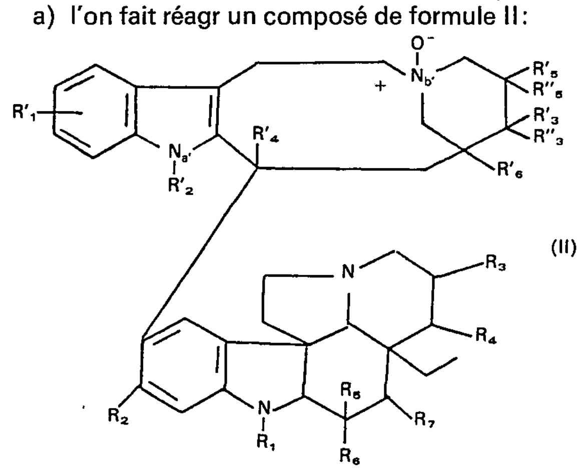

(II)

dans laquelle les substituants ont la signification donnée précédemment, en présence d'un réactif formateur d'ion immonium;

b) l'on traite le produit obtenu par un ou plusieurs réactifs nucléophiles choisis parmi les solvants organiques en présence ou non d'eau et un alcool, un phénol, un thiol, une amine, un ion cyanure, un ion hydrure ou l'ammoniaque, et on sépare le composé de formule I.

5. Procédé selon la revendication 4, caractérisé en ce que le réactif formateur d'ion immonium est un halogénure, anhydride ou sel d'acide organique ou inorganique.

6. Procédé selon la revendication 5, caractérisé en ce que le réactif formateur d'ion immonium est un halogénure, anhydride ou sel d'acide carboxylique halogéné.

7. Procédé selon la revendication 6, caractérisé en ce que le réactif formateur d'ion immonium est l'anhydride trifluoroacétique.

8. Procédé selon l'une des revendications 4 à 7, caractérisé en ce que l'étape a est conduite dans un solvant anhydre.

9. Procédé selon la revendication 7, caractérisé en ce que le solvant anhydre est un solvant chloré.

10. Procédé selon la revendication 9, caractérisé en ce que le solvant chloré est le chlorure de méthylène, le dichloroéthane ou le chloroforme.

11. Procédé selon l'une des revendications 4 à 10, caractérisé en ce que l'étape a est conduite à une température comprise entre −5 et +5°C.

12. Procédé selon l'une des revendications 4 à 10, caractérisé en ce que l'étape b est effectuée en traitant le produit de l'étape a par un mélange de solvant organique en présence ou non d'eau.

13. Procédé selon l'une des revendications 4 à 12, caractérisé en ce que le mélange réactionnel de l'étape a peut être amené à sec avant l'étape b.

14. Procédé selon l'une des revendications 4 à 13, caractérisé en ce que le composé de formule I est séparé du mélange réactionnel de l'étape b par extraction par un solvant chloré et chromatographie.

15. Procédé de préparation des composés selon l'une des revendications 1 à 3, caractérisé en ce que l'on fait réagir un composé de formule IV:

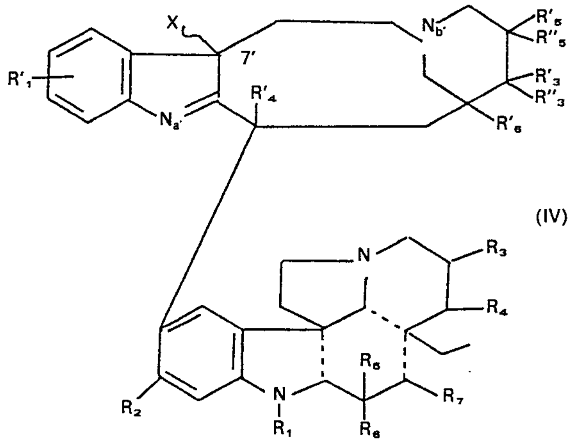

(IV)

dans laquelle les substituants ont la signification donnée précédemment et X représente un halogène, un radical hydroxy, hydroperoxy ou alcanoyloxy, en présence d'un réactif nucléophile choisi parmi les solvants organiques en présence ou non d'eau et un alcool, un phénol, un thiol, une amine, un ion cyanure, un ion hydrure, l'ammoniaque.

16. Procédé selon la revendication 15, caractérisé en ce que le solvant organique pourra être choisi parmi le méthanol, l'éthanol, le chloroforme, le dichlorométhane, le tétrahydrofuranne, le dioxanne, le diméthylformamide, le diméthylsulfoxyde, l'hexaméthylphosphorotriamide ou l'acétonitrile.

17. A titre de médicament nouveau, composé selon l'une des revendications 1 à 3.

18. Composition pharmaceutique contenant, à titre de principe actif, au moins un composé selon l'une des revendications 1 à 3.

## Patentansprüche

1. Verbindungen der allgemeinenen Formel

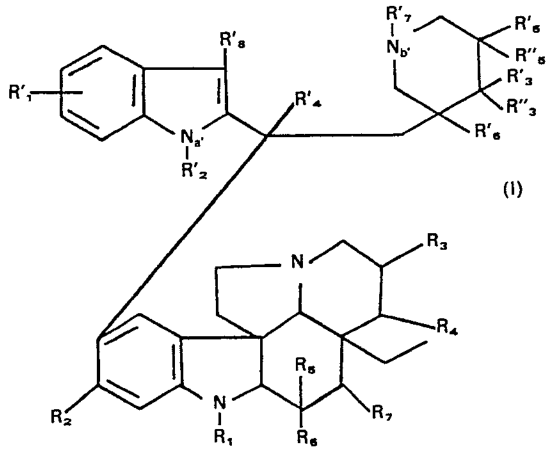

(I)

in der

$R'_1$ für Wasserstoff oder eine $C_1$ bis $C_5$-Alkoxy-, Acetyl-, Propionyl- oder Formyl-, Halogenacetyl- oder Halogenpropionylgruppe steht,

$R'_2$ für Wasserstoff oder eine $C_1$ bis $C_5$-Alkylgruppe steht,

$R'_3$ und $R''_3$ gleich oder verschieden sind, und für Wasserstoff oder eine Hydroxyl-, Acetyloxy- oder Propionyloxygruppe stehen, oder $R'_3$ und $R''_3$ gemeinsam eine Carbonylgruppe, oder $R'_3$ und $R'_5$ gemeinsam eine Epoxybrücke oder eine Doppelbindung bilden,

$R'_4$ für Wasserstoff oder eine ($C_1$ bis $C_5$-Alkyl)-oxycarbonyl-, Hydroxymethyl-, Acetyloxymethyl-, Propionyloxymethyl- oder Acetamidogruppe steht,

$R'_5$ und $R''_5$ gleich oder verschieden sind, und für Wasserstoff oder eine Hydroxyl-, Acetyloxy-, Propionyloxy-, Äthyl- oder Hydroxy-2-äthylgruppe stehen,

$R'_6$ für Wasserstoff oder eine Äthyl-, 2-Hydroxyäthyl- oder Acetylgruppe steht,

$R'_7$ und $R'_8$ gleich oder verschieden sind, und für Wasserstoff oder eine Methyl-, Hydroxymethylen-, $C_2$ bis $C_5$-Alkoxymethylen-, Aryloxymethylen-, Aminomethylen-, $C_1$ bis $C_5$-Monoalkyl- und di($C_1$ bis $C_5$-Alkyl)aminomethylen-, Arylaminomethylen-, Hydroxyäthylaminomethylen-, ($C_1$ bis $C_5$-Alkyl)thiomethylen-, Arylthiomethylen-, Cyanmethylen-, Acetyl- oder Propionylmethylengruppe stehen, unter der Voraussetzung, dass $R'_7$ und $R'_8$ zusammen nicht die Hydroxymethylengruppe bilden, und die Arylgruppen monozyklisch sind und 5 oder 6 Atome enthalten,

$R_1$ für Wasserstoff oder eine $C_1$ bis $C_5$-Alkyl-, Formyl-, Acetyl- oder Propionylgruppe steht,

$R_2$ für Wasserstoff oder eine $C_1$ bis $C_5$-Alkoxygruppe steht,

$R_3$ für Wasserstoff eine Hydroxyl-, Acetyloxy- oder Propionyloxygruppe steht, oder $R_3$ und $R_4$ gemeinsam eine Epoxybrücke oder eine Doppelbindung sind,

$R_4$ für Wasserstoff oder eine Hydroxyl-, Acetyloxy- oder Propionyloxygruppe steht, oder $R_4$ und $R_5$ gemeinsam eine Epoxybrücke sind,

$R_6$ für eine ($C_1$ bis $C_5$-Alkyl)oxycarbonyl-, Hydrazido-, Acetamido-, Hydroxymethyl-, Acetyloxymethylen- oder Propionyloxymethylengruppe steht, und

$R_5$ und $R_7$ für Wasserstoff oder eine Hydroxy-,. Acetyloxy- oder Propionyloxygruppe stehen, ebenso wie deren Säureadditionssalze und deren quaternäre Amoniumsalze.

2. Verbindungen nach dem Anspruch 1 gemäss der Formel (Ia):

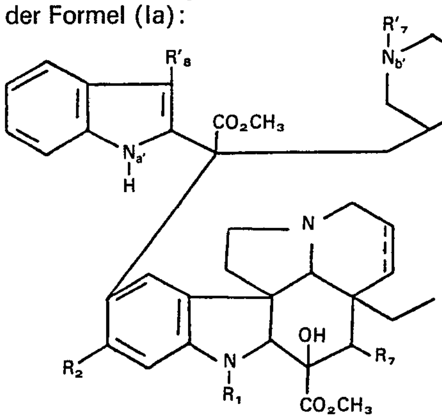

(Ia)

in der

$R'_3$ für Wasserstoff oder eine Hydroxygruppe steht,

$R'_5$ für Wasserstoff oder eine Hydroxygruppe steht,

$R'_3$ und $R'_5$ gemeinsam eine Epoxybrücke oder eine Doppelbindung sind,

$R''_5$ für Wasserstoff oder eine Äthylgruppe steht,

$R'_7$ und $R'_8$ gleich oder verschieden sind, und für Wasserstoff oder eine Methyl-, Hydroxymethylen-, ($C_1$ bis $C_5$-Alkoxy)methylen-, Aryloxymethylen-, Aminomethylen-, ($C_1$ bis $C_5$-Monoalkyl) oder ($C_1$ bis $C_5$-Dialkyl)aminomethylen-, ($C_1$ bis $C_5$-Acyl)methylen-, Arylaminomethylen-, Hydroxyäthylaminomethylen-, ($C_1$ bis $C_5$-Alkyl)thiomethylen-, Arylthiomethylen-, Cyanmethylengruppen stehen, vorausgesetzt, dass $R'_7$ und $R'_8$ gemeinsam nicht für die Hydroxymethylengruppe stehen, und die Arylgruppen monozyclisch sind und 5 oder 6 Atome enthalten,

$R_1$ für Wasserstoff oder eine $C_1$ bis $C_5$-Alkyl-, Formyl- oder Acylgruppe steht,

$R_2$ für Wasserstoff oder eine $C_1$ bis $C_5$-Alkoxygruppe steht, und

$R_7$ für Alkanoyloxygruppe steht,

wobei die gestrichelte Linie gegebenenfalls für eine Doppelbindung steht,

sowie deren entsprechenden Salze.

3. Verbindungen gemäss den Formeln (Ia1) bis (Ia5):

5',6'-Seco-5'-cyan-6'-methoxyanhydrovinblastin,

5',6'-Secoanhydrovinblastin,

5',6'-Seco-6'-methoxyanhydrovinblastin,

5',6'-Seco-5'-cyan-6'-methoxy-20'-deoxyleurosidin, und

5',6'-Seco-5'-dimethyl-6'-hydroxy-20'-deoxyleurosidin.

4. Verfahren zur Herstellung der Verbindungen gemäss dem Anspruch 1, dadurch gekennzeichnet, dass

a) man eine Verbindung der Formel (II):

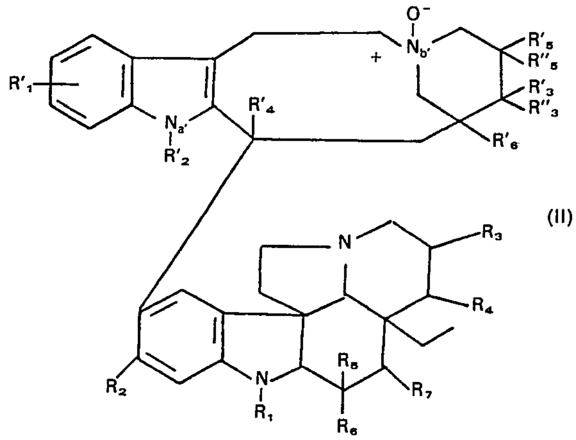

(II)

in Gegenwart eines reaktiven Immoniumionenbildners umsetzt, wobei in der Formel die Substituenten die vorgegebene Bedeutung besitzen;

b) man das erhaltene Produkt mit einem oder mehreren nukleophilen Reagentien wie organischen Lösungsmitteln in Ab- oder Anwesenheit von Wasser und einem Alkohol, Phenol, Thiol, Amin, einem Cyanidion, einem Hydridion oder Ammoniak behandelt und die Verbindung gemäss Formel I abtrennt.

5. Verfahren nach dem Anspruch 4, dadurch gekennzeichnet, dass der reaktive Immoniumionbildner ein Halogenid, Anhydrid oder Salz einer organischen oder anorganischen Säure ist.

6. Verfahren nach dem Anspruch 5, dadurch gekennzeichnet, dass der reaktive Immoniumionbildner ein Halogenid, Anhydrid oder Salz einer halogenierten Carbonsäure ist.

7. Verfahren nach dem Anspruch 6, dadurch gekennzeichnet, dass der reaktive Immoniumionbildner Trifluoressigsäureanhydrid ist.

8. Verfahren nach einem der Ansprüche 4 bis 7, dadurch gekennzeichnet, dass der Verfahrensschritt a in wasserfreiem Lösungsmittel durchgeführt wird.

9. Verfahren nach dem Anspruch 7, dadurch gekennzeichnet, dass das wasserfreie Lösungsmittel ein chloriertes Lösungsmittel ist.

10. Verfahren nach dem Anspruch 9, dadurch gekennzeichnet, dass das chlorierte Lösungsmittel Methylenchlorid, Dichloräthan oder Chloroform ist.

11. Verfahren nach einem der Ansprüche 4 bis 10, dadurch gekennzeichnet, dass die Verfahrensstufe a bei einer Temperatur zwischen −5°C und +5°C durchgeführt wird.

12. Verfahren nach einem der Ansprüche 4 bis 10, dadurch gekennzeichnet, dass der Verfahrensschritt b durchgeführt wird, indem man das Produkt aus dem Verfahrensschritt a mit einem organischen Lösungsmittelgemisch in Ab- oder Anwesenheit von Wasser behandelt.

13. Verfahren nach einem der Ansprüche 4 bis 12, dadurch gekennzeichnet, dass man die Reaktionsmischung aus der Verfahrensstufe a vor der im Verfahrensschritt b trocknet.

14. Verfahren nach einem der Ansprüche 4 bis 13, dadurch gekennzeichnet, dass die Verbindung gemäss der Formel (I) aus der Reaktionsmischung der Verfahrensstufe b durch Extraktion mit einem chlorierten Lösungsmittel und chromatographisch abtrennt.

15. Verfahren zur Herstellung von Verbindungen gemäss einem der Ansprüche 1 bis 3, dadurch gekennzeichnet, dass man eine Verbindung der Formel (IV):

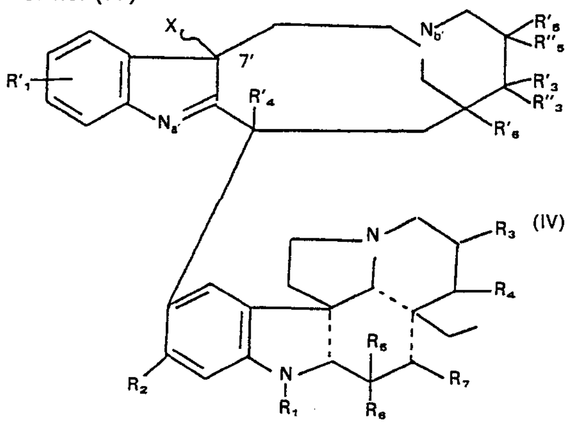

(IV)

in der die Substituenten die vorgegebene Bedeutung besitzen, und X für Halogen, eine Hydroxygruppe, Hydroperoxy- oder Alcanoyloxygruppe steht, in Gegenwart eines nukleophilen Reagenz, nämlich organischen Lösungsmitteln in Gegenwart oder Abwesenheit von Wasser und einem Alkohol, Phenol, Thiol, Amin, Cyanidion, Hydridion, Ammoniak umsetzt.

16. Verfahren nach dem Anspruch 15, dadurch gekennzeichnet, dass man ein organisches Lösungsmittel verwendet, dass man aus den Lösungsmitteln Methanol, Äthanol, Chloroform, Dichlormethan, Tetrahydrofuran, Dioxan, Dimethylformamid, Dimethylsulfoxid, Hexamethylphosphortriamid oder Acetonitril auswählen kann.

17. Eine Verbindung nach einem der Ansprüche 1 bis 3 als neues Arzneimittel.

18. Pharmazeutische Zusammensetzung mit Gehalt an mindestens einer Verbindung gemäss einem der Ansprüche 1 bis 3 als aktive Wirkungskomponente.

## Claims

1. Compounds corresponding to the general formula:

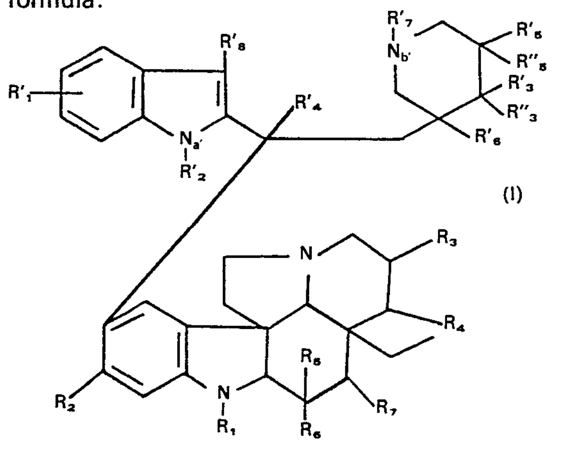

(I)

wherein

$R'_1$ represents a hydrogen atom or a $C_1$-$C_5$ alkoxy, acetyl, propionyl or formyl, halo-acetyl or halo-propionyl radical,

$R'_2$ represents a hydrogen atom or a $C_1$-$C_5$ alkyl radical,

$R'_3$ and $R''_3$ which may be the same or different, represent a hydrogen atom or a hydroxyl, acetyloxy or propionyloxy radical, or $R'_3$ and $R''_3$ together form a carbonyl group, or $R'_3$ and $R'_5$ together form an epoxy bridge or a double bond,

$R'_4$ represents a hydrogen atom or a ($C_1$-$C_5$ alkyl)oxycarbonyl, hydroxymethyl, acetyloxymethyl, propionyloxymethyl or acetamido radical,

$R'_5$ and $R''_5$ which may be the same or different, represent a hydrogen atom or a hydroxyl, acetyloxy, propionyloxy, ethyl or 2-hydroxyethyl radical,

$R'_6$ represents a hydrogen atom or an ethyl, 2-hydroxyethyl or acetyl radical,

$R'_7$ and $R'_8$ which may be the same or different, represent a hydrogen atom or a methyl, hydroxymethylene, $C_2$-$C_5$ alkoxymethylene, aryloxymethylene, aminomethylene, $C_1$-$C_5$ monoalkyl- and di($C_1$-$C_5$alkyl)-aminomethylene, arylaminomethylene, hydroxyethyl aminomethylene, ($C_1$-$C_5$ alkyl)thiomethylene, arylthiomethylene, cyanomethylene, acetyl- or propionylmethylene radical, provided that $R'_7$ and $R'_8$ do not together represent the hydroxymethylene radical, the aryl radicals being monocyclic and containing 5 or 6 atoms,

$R_1$ represents a hydrogen atom or a $C_1$-$C_5$ alkyl, formyl, acetyl or propionyl radical,

$R_2$ represents a hydrogen atom or a $C_1$-$C_5$ alkoxy radical,

$R_3$ represents a hydrogen atom or a hydroxyl, acetyloxy or propionyloxy radical, or $R_3$ and $R_4$ together form an epoxy bridge or a double bond,

$R_4$ represents a hydrogen atom or a hydroxyl, acetyloxy or propionyloxy radical, or $R_4$ and $R_5$ together form an epoxy bridge,

$R_6$ represents a ($C_1$-$C_5$ alkyl)oxycarbonyl, hydrazido, acetamido, hydroxymethyl, acetyloxymethylene or propionyloxymethylene radical, and

$R_5$ and $R_7$ represent a hydrogen atom or a hydroxide, acetyloxy or popionyloxy radical,

and their addition salts with acids and their quaternary ammonium salts.

2. Compounds according to claim 1 corresponding to formula Ia:

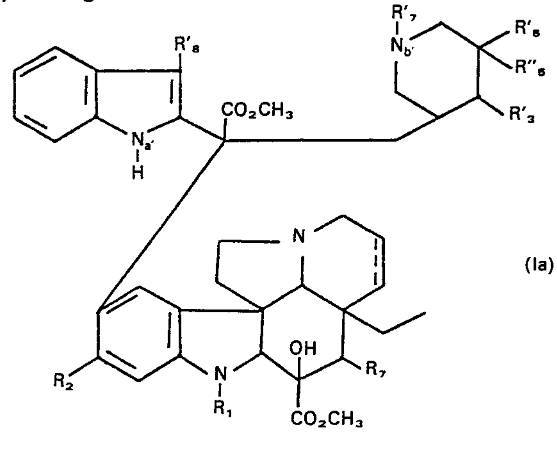

(Ia)

wherein

$R'_3$ represents a hydrogen atom or a hydroxy radical,

$R'_5$ represents a hydrogen atom or a hydroxy radical,

$R'_3$ and $R'_5$ together represent an expoxy bridge or a double bond,

$R''_5$ represents a hydrogen atom or an ethyl radical,

$R'_7$ and $R'_8$ which may be the same or different, represent a hydrogen atom or a methyl, hydroxymethylene, ($C_1$-$C_5$ alkoxy)methylene, aryloxymethylene, aminomethylene, ($C_1$-$C_5$ monoalkyl)- or ($C_1$-$C_5$ dialkyl)aminomethylene, ($C_1$-$C_5$ acyl)-methylene, arylaminomethylene, hydroxyethylaminomethylene, ($C_1$-$C_5$ alkyl)thiomethylene, arylthiomethylene, cyanomethylene radical, provided that $R'_7$ and $R'_8$ do not together represent the hydroxymethylene radical, the aryl radicals being monocyclic and containing 5 or 6 atoms,

$R_1$ represents a hydrogen atom or a $C_1$-$C_5$ alkyl, formyl or acyl radical,

$R_2$ represents a hydrogen atom or a $C_1$-$C_5$ alkoxy radical, and

$R_7$ represents an alkylanoyloxyl radical,

the dashed line represents a possible double bond, and the corresponding salts.

3. The compounds corresponding to formulae Ia1 to Ia5:

5',6'-seco-5'-cyano-6'-methoxy- anhydrovinblastine,

5',6'-seco-anhydrovinblastine,

5',6'-seco-6'-methoxy- anhydrovinblastine,

5',6'-seco-5'-cyano-6'-methoxy-20'-deoxyleurosidine,

5',6'-seco-5'-dimethyl-6'-hydroxy-20'-deoxyleurosidine.

4. A process for the preparation of the compounds according to claim 1, characterised in that:

a) a compound corresponding to formula II:

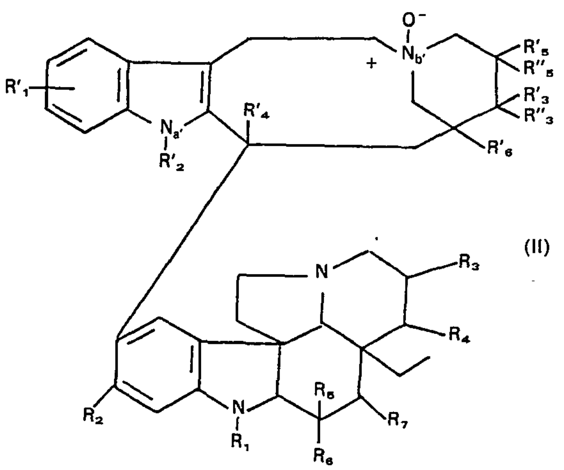

(II)

wherein the substituents are as defined above, is reacted in the presence of an immonium ion-forming reagent;

b) the resulting product is treated with one or more nucleophilic reagents selected from among: organic solvents in the presence, or in the absence, of water and an alcohol, a phenol, a thiol, an amine, a cyanide ion, a hydride ion or ammonia, and a compound corresponding to formula I is separated.

5. A process according to claim 4, characterised in that the immonium ion-forming reagent is a halide, an anhydride or a salt of an organic or inorganic acid.

6. A process according to claim 5, characterised in that the immonium ion-forming reagent is a halide, an anhydride or a salt of a halogenated carboxylic acid.

7. A process according to claim 6, characterised in that the immonium ion-forming agent is trifluoroacetic anhydride.

8. A process according to one of claims 4 to 7, characterised in that step a) is carried out in an anhydrous solvent.

9. A process according to claim 7, characterised in that the anhydrous solvent is a chlorinated solvent.

10. A process acording to claim 9, characterised in that the chlorinated solvent is methylene chloride, dichloroethane or chloroform.

11. A process according to one of claims 4 to 10, characterised in that step a) is carried out at a temperature of from −5 to +5° C.

12. A process according to one of claims 4 to 10, characterised in that step b) is carried out by treating the product of step a) with a mixture of organic solvent in the presence, or in the absence, of water.

13. A process according to one of claims 4 to 12, characterised in that the reaction mixture of step a) may be dried before step b).

14. A process according to one of claims 4 to 13, characterised in that the compound corresponding to formula I is separated from the reaction mixture of step b) by extraction using a chlorinated solvent and chromatography.

15. A process for the preparation of compounds according to one claims 1 to 3, characterised in that a compound corresponding to formula IV:

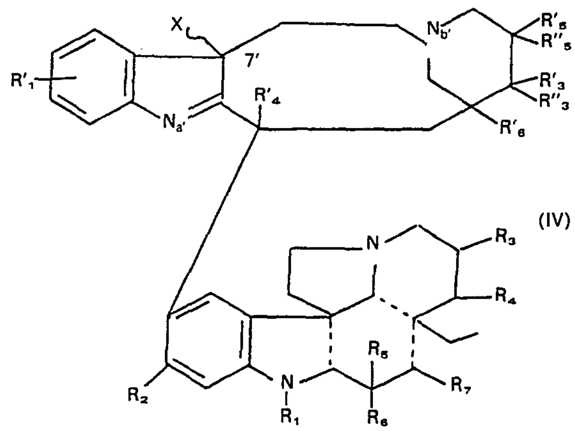

(IV)

wherein the substituents are as defined above, and X represents a halogen, a hydroxy, hydroperoxy or alkanoyloxy radical, is reacted in the presence of a nucleophilic reagent selected from among: organic solvents in the presence, or in the absence, of water and an alcohol, a phenol, a thiol, an amine, a cyanide ion, a hydride ion or ammonia.

16. A process according to claim 15, characterised in that the organic solvent may be selected from among methanol, ethanol, chloroform, dichloromethane, tetrahydrofuran, dioxane, dimethylformamide, dimethylsulphoxide, hexamethylphosphorotriamide or acetonitrile.

17. A compound according to one of claims 1 to 3 as a new medicament.

18. A pharmaceutical composition containing at least one compound according to one of claims 1 to 3 as an active principle.